# EUROPÄISCHE PATENTANMELDUNG

(11) **EP 2 213 370 A2**
(43) Veröffentlichungstag der Anmeldung: **04.08.2010**
(21) Anmeldenummer: 10150190.6
(22) Anmeldetag: 06.01.2010
(51) Int. Cl.: B01J 23/76, B01J 23/881, B01J 37/03, C07C 45/38

(54) **Verfahren zur Herstellung eines Katalysators fuer die Oxidation von Methanol zu Formaldehyd**

(30) Priorität: 09.01.2009 EP 09150278
(71) Anmelder: BASF SE, 67056 Ludwigshafen (DE)
(72) Erfinder: Abdallah, Radwan, 67063, Ludwigshafen (DE); Hibst, Hartmut, 69198, Schriesheim (DE); Schindler, Goetz-Peter, 67071, Mannheim (DE); Johann, Thorsten, 67063, Ludwigshafen (DE); Borchert, Holger, 67591, Offstein (DE); Zuelke, Juergen, 67346, Speyer (DE)
(74) Vertreter: Isenbruck, Günter

(57) **Zusammenfassung**

Die vorliegende Erfindung betrifft ein Verfahren zur Herstellung eines Oxidgemisches enthaltend wenigstens eine Verbindung der allgemeinen Formel (1) Feₓ(M¹O_{y})_{z} und wenigstens eine Verbindung der allgemeinen Formel (II) M¹ₘOₙ, ein solches Oxidgemisch, die Verwendung dieses Oxidgemisches als Katalysator bei der Herstellung von Formaldehyd durch Oxidation von Methanol, sowie ein Verfahren zur Herstellung von Formaldehyd durch Oxidation von Methanol, **dadurch gekennzeichnet, dass** ein solches Oxidgemisch als Katalysator eingesetzt wird.
M¹ ist hierbei ein Metall ausgewählt aus der Gruppe bestehend aus Mo, V, Cu, Cr, Co, P, Si, B, S, Na und Mischungen davon.

## Beschreibung

Die vorliegende Erfindung betrifft ein Verfahren zur Herstellung eines Oxidgemisches enthaltend wenigstens eine Verbindung der allgemeinen Formel Feₓ(M¹O_{y})_{z} und wenigsten eine Verbindung der allgemeinen Formel M¹ₘOₙ, wobei M¹ ein Element ist, ausgewählt aus der Gruppe bestehend aus Mo, V, Cu, Cr, Co, P, Si, B, S, Na und Mischungen davon und x, y, z, m und n ganze Zahlen darstellen, so dass die genannten Verbindungen jeweils ladungsneutral sind, das Oxidgemisch herstellbar nach dem erfindungsgemäßen Verfahren und die Verwendung dieses Oxidgemisches als Katalysator bei der Herstellung von Formaldehyd durch Oxidation von Methanol.

Oxidgemische, welche eine katalytische Aktivität für die Herstellung von Formaldehyd durch Oxidation von Methanol aufweisen, und Verfahren zu deren Herstellung sind aus dem Stand der Technik bereits bekannt.

WO 02/22539 offenbart ein Verfahren zur Herstellung von Formaldehyd durch Oxidation von Methanol, welches in Gegenwart eines Molybdän-Eisen-Katalysators durchgeführt wird. Dazu werden gemäß WO 02/22539 verschiedene Molybdate eingesetzt, enthaltend Mangan, Chrom, Zink, Aluminium oder Zirconium.

EP 0 591 572 A1 offenbart Katalysatoren in Granulatform, welche besonders für die oxidative Dehydrierung von Methanol zur Erzeugung von Formaldehyd eingesetzt werden. Diese Katalysatoren sind bevorzugt gemischte Eisen-Molybdän-Oxide, welche durch Coextrusion in eine bestimmte Geometrie gebracht werden.

EP 0 732 146 A1 offenbart tablettierte Katalysatoren bzw. Katalysatorträger enthaltend Eisen-(III)-molybdat und Molybdän-(VI)-oxid. Diese tablettierten Katalysatoren können für eine Vielzahl verschiedener Reaktionen verwendet werden, beispielsweise als Katalysatoren für die Oxidation von Methanol zu Formaldehyd.

EP 1 645 332 A1 offenbart ebenfalls einen Katalysator, der durch Tablettierung erhalten wird. Dieser enthält als aktive Verbindungen Eisen-(III)-molybdat und Molybdän-(VI)-oxid, wobei der Hauptanteil des Porenvolumens in den Katalysatorteilchen von Poren bereitgestellt wird, die einen Radius zwischen 1000 und 2000 Angström aufweisen.

DE 2 145 851 offenbart ein Verfahren zur Herstellung eines Katalysators, der für die Oxidation von Methanol zu Formaldehyd eingesetzt werden kann. Dieser Katalysator enthält Molybdän-(VI)-oxid und Eisen-(III)-oxid. Zusätzlich enthält der Katalysator gemäß DE 2 145 851 Titandioxid. Das Oxidgemisch enthaltend Molybdän-(VI)-oxid und Eisen-(III)-oxid wird durch gemeinsames Fällen aus wässriger Lösung erhalten, dieses Präzipitat wird getrocknet und calciniert.

Die aus dem Stand der Technik bekannten Verfahren zur Herstellung von Oxidgemischen enthaltend Eisenmolybdat und Molybdänoxid bzw. Eisen- und Molybdänoxide zeichnen sich dadurch aus, dass aus wässrigen Lösungen die entsprechenden Oxidgemische durch Ausfällen erhalten werden, diese Oxidgemische werden getrocknet und anschließend calciniert.

Aufgabe der vorliegenden Erfindung ist es, ein Verfahren zur Herstellung von Oxidgemischen, die als Katalysatoren für die oxidative Herstellung von Formaldehyd aus Methanol geeignet sind, bereitzustellen, welches entsprechende Oxidgemische liefert, die sich durch eine erhöhte Stabilität und vor allem eine erhöhte Selektivität in dem genannten Verfahren auszeichnen. Eine weitere Aufgabe der vorliegenden Erfindung ist es, ein solches Verfahren bereitzustellen, welches sich durch einfachen apparativen Aufbau auszeichnet.

Diese Aufgaben werden gelöst durch ein Verfahren zur Herstellung eines Oxidgemisches enthaltend wenigstens eine Verbindung der allgemeinen Formel (I)

Feₓ(M¹O_{y})_{z} (I)

und wenigstens eine Verbindung der allgemeinen Formel (II)

M¹ₘOₙ (II),

in denen M¹, x, y, z, m und n die folgenden Bedeutungen haben:
- M¹: Element ausgewählt aus der Gruppe bestehend aus Mo, V, Cu, Cr, Co, P, Si, B, S, Na und Mischungen davon,
- x: ganze Zahl von 1 bis 3,
- y: ganze Zahl von 1 bis 6,
- z: ganze Zahl von 1 bis 4,
- m: ganze Zahl von 1 bis 3 und
- n: ganze Zahl von 1 bis 5,
wobei x, y, z, m und n so gewählt sind, dass die Verbindungen der allgemeinen Formeln (I) und (II) jeweils ladungsneutral sind, umfassend die Schritte:
(A) Bereitstellen einer Lösung von Verbindungen enthaltend wenigstens Eisen- und M¹-Kationen in einem geeigneten Lösungsmittel,
(B) Herstellen eines Oxidgemisches enthaltend wenigstens eine Verbindung der allgemeinen Formel (I) und wenigstens eine Verbindung der allgemeinen Formel (II) durch gemeinsames Ausfällen der Verbindungen aus der in Schritt (A) hergestellten Lösung,
(C) Abtrennen des in Schritt (B) erhaltenen Oxidgemisches von dem Lösungsmittel und
(D) Calcinieren des in Schritt (C) erhaltenen Oxidgemisches bei einer Temperatur von 80 bis 550 °C in einem sauerstoffhaltigen Gas,
wobei sich aus den in Schritt (A) eingesetzten Verbindungen in Schritt (B) und/oder (D) wenigstens eine Verbindung G bildet, die von den Verbindungen der allgemeinen Formeln (I) und (II) unterschiedlich ist, und in Schritt (D) das molare Verhältnis von Sauerstoff zu der wenigstens einen Verbindung G n(O₂)/n(G) größer als 0,10 ist.

Das erfindungsgemäße Verfahren zeichnet sich dadurch aus, dass Oxidgemische zugänglich sind, welche besonders für den Einsatz als Katalysatoren bei der Herstellung von Formaldehyd durch oxidative Behandlung von Methanol geeignet sind.

Das erfindungsgemäß herstellbare Oxidgemisch enthält wenigstens eine Verbindung der allgemeinen Formel (I)

Feₓ(M¹O_{y})_{z} (I),

wobei M¹ ein Element ist, ausgewählt aus der Gruppe bestehend aus Mo, V, Cu, Cr, Co, P, Si, B, S, Na und Mischungen davon, bevorzugt Mo, x für eine ganze Zahl von 1 bis 3, bevorzugt 2 steht, y für eine ganze Zahl von 1 bis 6, bevorzugt 4 steht und z für eine ganze Zahl von 1 bis 4, bevorzugt 3 steht, wobei x, y und z jeweils so gewählt sind, dass die Verbindung der allgemeinen Formel (I) ladungsneutral ist.

In einer bevorzugten Ausführungsform wird durch das erfindungsgemäße Verfahren ein Oxidgemisch gebildet, in der in der Verbindung der allgemeinen Formel (I) Fe in der Oxidationsstufe +3 vorliegt. In einer besonders bevorzugten Ausführungsform liegt in dem erfindungsgemäßen Oxidgemisch kein, d. h. weniger als 0,5 Gew.-%, Eisen in der Oxidationsstufe +2, beispielsweise als FeMoO₄, vor.

In einer bevorzugten Ausführungsform ist M¹ ein Metall ausgewählt aus der Gruppe bestehend aus Mo, V, Cu, Cr, Co und Mischungen davon. Gegebenenfalls liegt zusätzlich ein Element ausgewählt aus der Gruppe bestehend aus P, Si, B, S, Na und Mischungen davon neben wenigstens einem der genannten Metalle M¹ in den Verbindungen der allgemeinen Formeln (I) und/oder (II) vor. Das Element ausgewählt aus der Gruppe bestehend aus P, Si, B, S, Na und Mischungen davon liegt dabei in den Verbindungen der allgemeinen Formeln (I) und (II) unabhängig voneinander in einer Menge von 0,001 bis 20 Gew.-%, bevorzugt 0,01 bis 10 Gew.-%, jeweils bezogen auf die Gesamtmenge aus wenigstens einem Metall M¹ und wenigstens einem Element M¹, vor.

In einer besonders bevorzugten Ausführungsform ist die wenigstens eine Verbindung der allgemeinen Formel (I) Fe₂(MoO₄)₃.

Das erfindungsgemäß herstellbare Oxidgemisch enthält des Weiteren wenigstens eine Verbindung der allgemeinen Formel (II)

M¹ₘOₙ (II),

wobei m eine ganze Zahl von 1 bis 3, bevorzugt 1, und n eine ganze Zahl von 1 bis 5, bevorzugt 3, bedeuten, wobei m und n jeweils so gewählt sind, dass die Verbindung der allgemeinen Formel (II) ladungsneutral ist. In einer besonders bevorzugten Ausführungsform bedeutet m 1 und n 3, so dass in einer besonders bevorzugten Ausführungsform die wenigstens eine Verbindung der allgemeinen Formel (II) MoO₃ ist.

In einer besonders bevorzugten Ausführungsform des erfindungsgemäßen Verfahrens ist die Verbindung der allgemeinen Formel (I) Fe₂(MoO₄)₃ und die Verbindung der allgemeinen Formel (II) ist MoO₃.

Das Atomverhältnis Molybdän zu Eisen in dem erfindungsgemäß herstellbaren Oxidgemisch enthaltend wenigstens eine Verbindung der allgemeinen Formel (I) und wenigstens eine Verbindung der allgemeinen Formel (II) beträgt im Allgemeinen 1:1 bis 7:1, bevorzugt 3:1 bis 6:1, besonders bevorzugt 5:1.

Rein formal kann die Verbindung Fe₂(MoO₄)₃ auch als eine Mischung aus einem Anteil Fe₂O₃ und drei Anteilen MoO₃ betrachtet werden. Erfindungsgemäß besonders bevorzugt herstellbare Oxidgemische enthalten formal betrachtet vorzugsweise 65 bis 95 Gew.-% MoO₃ und 5 bis 35 Gew.-% Fe₂O₃, jeweils bezogen auf die Summe aus MoO₃ und Fe₂O₃.

Im Folgenden werden die einzelnen Schritte des erfindungsgemäßen Verfahrens detailliert beschrieben:
Schritt (A):
   Schritt (A) des erfindungsgemäßen Verfahrens umfasst das Bereitstellen einer Lösung von Verbindungen enthaltend wenigstens Eisen- und M¹-Kationen in einem geeigneten Lösungsmittel.
   Erfindungsgemäß können alle dem Fachmann bekannten Verbindungen in Schritt (A) zur Herstellung einer entsprechenden Lösung in einem geeigneten Lösungsmittel verwendet werden, welche in dem verwendeten Lösungsmittel in ausreichender Menge löslich sind. Beispiele für geeignete Verbindungen, die Eisen-Kationen, bevorzugt Eisen(III)-Kationen enthalten, sind Eisen-(III)-nitrat Fe(NO₃)₃, Eisen-(III)-chlorid FeCl₃ und Mischungen davon. Eisen-(III)-nitrat wird bevorzugt als Hydrat eingesetzt, beispielsweise als Fe(NO₃)₃ · 9 H₂O.
   Geeignete Verbindungen, die M¹-Kationen enthalten, sind beispielsweise die entsprechenden Oxide, beispielsweise MoO₃, Ammoniummolybdate, wie (NH₄)₆(Mo₇O₂₄), bevorzugt als (NH₄)₆(Mo₇O₂₄) · 4 H₂O, falls M¹ Mo ist, oder V₂O₅, falls M¹ V ist.
   Die genannten Verbindungen enthaltend Eisen- und M₁-Kationen, insbesondere Mo-Kationen, werden in Schritt (A) in einem geeigneten Lösungsmittel gelöst, um eine entsprechende Lösung zu erhalten. Erfindungsgemäß sind alle Lösungsmittel, die dem Fachmann bekannt sind, geeignet, welche eine genügend große Löslichkeit der genannten Verbindungen ermöglichen. In einer besonders bevorzugten Ausführungsform wird Wasser als Lösungsmittel in Schritt (A) verwendet. In einer weiteren bevorzugten Ausführungsform wird in Schritt (A) eine wässrige Ammoniaklösung, beispielsweise 20 bis 30 gew.-%ig, verwendet.
   In einer besonders bevorzugten Ausführungsform, falls M¹ Mo ist, werden als Ausgangsverbindungen in Schritt (A) des erfindungsgemäßen Verfahrens MoO₃ in wässriger Ammoniaklösung oder wenigstens ein Ammoniummolybdat, beispielsweise Ammoniumheptamolybdat (NH₄)₆(Mo₇O₂₄), bevorzugt als Hydrat (NH₄)₆(Mo₇O₂₄) · 4 H₂O, in wässriger Lösung verwendet.
   In einer besonders bevorzugten Ausführungsform betrifft die vorliegende Erfindung das erfindungsgemäße Verfahren, wobei die in Schritt (A) hergestellte Lösung Fe(NO₃)₃ und wenigstens ein Ammoniummolybdat, beispielsweise Ammoniumheptamolybdat (NH₄)₆(Mo₇O₂₄), bevorzugt als Hydrat (NH₄)₆(Mo₇O₂₄) · 4 H₂O, enthält.
   Es ist erfindungsgemäß auch möglich, dass die insbesonders bevorzugten Lösungsmittel Wasser oder wässrige Ammoniaklösung wenigstens eine weitere Komponente, bevorzugt ein wasserlösliches, organisches Lösungsmittel, aufweist. Beispiele für erfindungsgemäß geeignete, wasserlösliche, organische Lösungsmittel sind Alkohole, beispielsweise Methanol, Ethanol, iso-Propanol, n-Propanol, iso-Butanol, tert-Butanol, n-Butanol, Ketone, beispielsweise Aceton, Ether, Dimethylsulfoxid (DMSO), Dimethylformamid (DMF), N-Methylpyrrolidon (NMP) und Mischungen davon.
   In einer bevorzugten Ausführungsform des erfindungsgemäßen Verfahrens werden in Schritt (A) des erfindungsgemäßen Verfahrens wenigstens zwei Lösungen bereitgestellt, wobei wenigstens eine erste Lösung bereitgestellt wird, die Eisen-Kationen enthält, besonders bevorzugt Eisen-(III)-Kationen, und wenigstens eine zweite Lösung bereitgestellt wird, die M¹-Kationen, insbesondere Molybdän-Kationen, ganz besonders bevorzugt Molybdän-(VI)-Kationen, enthält, und diese wenigstens zwei Lösungen zu einer Lösung vereinigt werden. Besonders bevorzugt ist die erste Lösung eine wässrige Lösung. Besonders bevorzugt ist die zweite Lösung eine wässrige Ammoniaklösung.
   Die in Schritt (A) des erfindungsgemäßen Verfahrens hergestellte(n) Lösung(en) können/kann nach allen dem Fachmann bekannten Verfahren erhalten werden, beispielsweise Vorlegen der zu lösenden Komponenten und Zugabe des Lösungsmittels, bzw. Vorlegen des entsprechenden Lösungsmittels und Zugabe der zu lösenden Komponenten. Schritt (A) des erfindungsgemäßen Verfahrens kann bei allen dem Fachmann für geeignet erscheinenden Temperaturen durchgeführt werden, beispielsweise Umgebungstemperatur bis 100 °C, bevorzugt 50 bis 80 °C.
   Die Konzentrationen der einzelnen Komponenten in der in Schritt (A) des erfindungsgemäßen Verfahrens hergestellten Lösung werden im Allgemeinen so eingestellt, dass die einzelnen Komponenten in den Mengen vorliegen, in denen sie in dem erfindungsgemäß herzustellenden Oxidgemisch vorliegen werden. Beispielsweise liegt die Eisen(III)-Kationen enthaltende Verbindung, bevorzugt Eisen(III)-nitrat, im Allgemeinen in einer Konzentration von 2 bis 25 Gew.-%, bevorzugt 5 bis 20 Gew.-%, jeweils bezogen auf die gesamte in Schritt (A) hergestellte Lösung, vor. Die M¹-Kationen enthaltende Verbindung, bevorzugt Ammoniumheptamolybdat, liegt im Allgemeinen in einer Konzentration von 20 bis 90 Gew.-%, bevorzugt 30 bis 80 Gew.-%, jeweils bezogen auf die gesamte in Schritt (A) hergestellte Lösung, vor.
   Das molare Verhältnis von Eisen(III)-Kationen zu M¹-Kationen n(Fe(III))/n(M¹) beträgt dabei im Allgemeinen 0,1 bis 2,0, bevorzugt 0,2 bis 1,6, besonders bevorzugt 0,3 bis 1,1, beispielsweise 0,35.
Schritt (B):
   Schritt (B) des erfindungsgemäßen Verfahrens umfasst das Herstellen eines Oxidgemisches enthaltend wenigstens eine Verbindung der allgemeinen Formel (I) und wenigstens eine Verbindung der allgemeinen Formel (II) durch gemeinsames Ausfällen dieser Verbindungen aus der in Schritt (A) hergestellten Lösung.
   Ausfällen bedeutet im Rahmen der vorliegenden Erfindung, dass das Löslichkeitsprodukt einer der in Lösung vorliegenden Verbindungen in dem Lösungsmittel, bevorzugt Wasser, überschritten ist, so dass so lange die entsprechende Verbindung als Feststoff aus der Lösung ausfällt, bis das Löslichkeitsprodukt in der Lösung wieder erreicht ist. Ein Fachmann weiß, wie die Konzentrationen einzustellen sind, um ein Ausfällen einer Verbindung aus einer Lösung zu bewirken.
   In einer bevorzugten Ausführungsform des erfindungsgemäßen Verfahrens werden in Schritt (A) zwei Lösungen hergestellt, die dann zu einer Lösung vereinigt werden, so dass das erfindungsgemäße Oxidgemisch enthaltend wenigstens eine Verbindung der allgemeinen Formel (I) und wenigstens eine Verbindung der allgemeinen Formel (II) ausfällt. Dieses Zusammengeben kann bei einer Temperatur von Umgebungstemperatur bis 100°C durchgeführt werden. Bevorzugt wird beim Zusammengeben der beiden Lösungen gerührt, so dass eine homogene Durchmischung der einzelnen Komponenten gewährleistet ist.
   Das Ausfallen des Oxidgemisches kann nach allen dem Fachmann bekannten Verfahren erfolgen, beispielsweise Erhöhung oder Erniedrigung des pH-Wertes, beispielsweise durch Zugabe wenigstens einer basischen oder sauren Verbindung, oder Erniedrigung der Temperatur der Mischung. In einer bevorzugten Ausführungsform findet das Ausfällen in Schritt (B) ohne weitere Maßnahmen, d. h. durch bloßes Herstellen der Lösung, beispielsweise durch Zusammengeben der unter Schritt (A) beschriebenen Löasungen, enthaltend die genannten Komponenten, statt.
   Nach bevorzugt vollständigem Ausfällen des erfindungsgemäß herstellbaren Oxidgemisches liegt eine Suspension des ausgefallenen Oxidgemischs in dem in Schritt (A) eingesetzten Lösungsmittel vor. Diese Aufschlämmung von Oxidgemisch in Lösungsmittel wird in einer bevorzugten Ausführungsform direkt in Schritt (C) eingesetzt.
Schritt (C):
   Schritt (C) des erfindungsgemäßen Verfahrens umfasst das Abtrennen des in Schritt (B) erhaltenen Oxidgemisches von dem Lösungsmittel. In einer bevorzugten Ausführungsform wird das erhaltene Oxidgemisch in Schritt (C) auch getrocknet, d. h. möglichst vollständig vom Lösungsmittel befreit.
   Erfindungsgemäß können in Schritt (C) des erfindungsgemäßen Verfahrens alle dem Fachmann bekannten Vorgehensweisen zum Abtrennen eines Feststoffes von einer Flüssigkeit verwendet werden, beispielsweise Filtrieren, Abdekantieren, Zentrifugieren und/oder Sprühtrocknen. Das Trocknen des erfindungsgemäß hergestellten Oxidgemischs erfolgt nach dem Fachmann bekannten Verfahren, beispielsweise Erwärmen auf eine Temperatur oberhalb Umgebungstemperatur, beispielsweise 40 bis 180 °C, gegebenenfalls verbunden mit einer Erniedrigung des Druckes auf unterhalb Atmosphärendruck, beispielsweise kleiner als 500 mbar, bevorzugt kleiner 100 mbar, beispielsweise 1 mbar.
   In einer besonders bevorzugten Ausführungsform erfolgt Schritt (C) des erfindungsgemäßen Verfahrens als Sprühtrocknung. Das Verfahren der Sprühtrocknung ist dem Fachmann bekannt. Vorteilhaft an einer Sprühtrocknung ist, dass in einem Schritt das gebildete Oxidgemisch von dem Lösungsmittel abgetrennt und gleichzeitig getrocknet wird.
   Das nach der Sprühtrocknung in Schritt (C) des erfindungsgemäßen Verfahrens erhaltene Oxidgemisch enthält gegebenenfalls noch Reste des Lösungsmittels. In einer bevorzugten Ausführungsform wird dieses Oxidgemisch weiter getrocknet, beispielsweise bei Temperaturen von 100 bis 200°C. Geeignete Vorrichtungen dazu sind dem Fachmann bekannt, beispielsweise ein Bandtrockner oder ein Muffelofen. Das in Schritt (C) des erfindungsgemäßen Verfahrens erhaltene trockene Oxidgemisch wird in einer bevorzugten Ausführungsform direkt in Schritt (D) des erfindungsgemäßen Verfahrens überführt. Trocken bedeutet im Rahmen der vorliegenden Erfindung, dass das Oxidgemisch nach Schritt (C) einen Gehalt an Lösungsmittel von bevorzugt weniger als 10 Gew.-%, besonders bevorzugt weniger als 5 Gew.-%, aufweist.
Schritt (D):
   Schritt (D) des erfindungsgemäßen Verfahrens umfasst das Calcinieren des in Schritt (C) erhaltenen Oxidgemisches bei einer Temperatur von 80 bis 550 °C in einem sauerstoffhaltigen Gas.

Aus den in Schritt (A) eingesetzten Verbindungen bildet sich erfindungsgemäß in Schritt (B) und/oder (D) wenigstens eine Verbindung G, die von den Verbindungen der allgemeinen Formeln (I) und (II) unterschiedlich ist. Es wurde nun gefunden, dass in Schritt (D) das molare Verhältnis von Sauerstoff zu der wenigstens einen Verbindung G n(O₂)/n(G) größer als 0,20 sein muss.

In Schritt (A) des erfindunsgemäßen Verfahrens wird eine Lösung bereitgestellt, die wenigstens Eisen- und M¹-Kationen enthält, wobei M¹ die oben genannten Bedeutungen hat. Aus den Verbindungen, die in Schritt (A) eingesetzt werden, die wenigstens Eisen- und M¹-Kationen enthalten, bilden sich in Schritt (B) des erfindungsgemäßen Verfahrens die Verbindungen der allgemeinen Formeln (I) und (II) und neben den Verbindungen der allgemeinen Formeln (I) und (II) auch wenigstens eine Verbindung G.

Welche Verbindung(en) als Verbindung G vorliegen, ist abhängig von den in Schritt (A) eingesetzten Ausgangsverbindungen. Für den besonders bevorzugten Fall, dass in Schritt (A) des erfindungsgemäßen Verfahrens Eisen(III)-nitrat Fe(NO₃)₃ als eisenhaltige Verbindung und wenigstens ein Ammoniummolybdat, beispielsweise (NH₄)₆Mo₇O₂₄ · 4 H₂O als molybdänhaltige Verbindungen eingesetzt werden, ist Verbindung G NH₄NO₃.

In Schritt (D) des erfindungsgemäßen Verfahrens ist das molare Verhältnis von Sauerstoff zu der wenigstens einen Verbindung G n(O₂)/n(G) im Allgemeinen größer als 0,10, bevorzugt größer als 0,20, besonders bevorzugt größer als 0,40, ganz besonders bevorzugt größer als 1,00, insbesondere größer als 1,80. Das molare Verhältnis von Sauerstoff zu der wenigstens einen Verbindung G n(O₂)/n(G) ist im Allgemeinen kleiner als 3,00, bevorzugt kleiner als 2,50.

Das für das erfindungsgemäße Verfahren wichtige molare Verhältnis von Sauerstoff zu der wenigstens einen Verbindung G kann insbesondere durch die Flussrate des sauerstoffhaltigen Gases in Schritt (D) eingestellt werden. Diese liegt im Allgemeinen bei wenigstens 25 NI/h, bevorzugt wenigstens 50 NI/h, besonders bevorzugt wenigstens 75 NI/h und insbesondere bevorzugt wenigstens 100 NI/h aufweist.

Durch die genannte Flussrate des sauerstoffhaltigen Gases und das damit verbundene molare Verhältnis von Sauerstoff zu der wenigstens einen Verbindung G werden erfindungsgemäß Oxidgemische erhalten, welche besonders gut als Katalysatoren bei der Herstellung von Formaldehyd durch oxidative Dehydrierung von Methanol eingesetzt werden können, da sie sich durch eine besonders hohe Stabilität und eine besonders hohe Selektivität bezüglich des gewünschten Produktes, beispielsweise bezüglich Formaldehyd aus Methanol, auszeichnen. Die durch das erfindungsgemäße Verfahren herstellbaren Oxidgemische weisen im Wesentlichen Eisen in der Oxidationsstufe +3, und kaum Eisen in der Oxidationsstufe +2, beispielsweise als FeMoO₄, auf. In einer bevorzugten Ausführungsform ist der Anteil an Eisen in der Oxidationsstufe +2 in den erfindungsgemäß hergestellten Oxidgemischen ≤ 0,5 Gew.-%. Dieser Umstand ist unter anderem dafür verantwortlich, dass die erfindungsgemäß hergestellten Oxidgemische besonders vorteilhaft als Katalysatoren bei der Herstellung von Formaldehyd durch Oxidation von Methanol eingesetzt werden können.

Im Allgemeinen erfolgt die Calcinierung in Schritt (D) des erfindunsgemäßen Verfahrens bei einer Temperatur von 80 bis 550°C. Das Verfahren der Calcinierung ist im Prinzip dem Fachmann bekannt.

In einer bevorzugten Ausführungsform wird das in Schritt (C) des erfindungsgemäßen Verfahrens erhaltene Oxidgemisch in Schritt (D) nicht bei einer konstanten, sondern bei verschiedenen Temperaturen behandelt. Beispielsweise wird das in Schritt (C) erhaltene Oxidgemisch zunächst in einer ersten Stufe auf eine Temperatur von 80 bis 160 °C, bevorzugt 90 bis 140 °C, beispielsweise für 10 bis 20 Stunden, erhitzt, dann in einem Zeitraum von beispielsweise 2 bis 20 Stunden auf eine Temperatur von 300 bis 550 °C, bevorzugt 400 bis 500 °C, gebracht und beispielsweise 1 bis 10 Stunden bei dieser Temperatur gehalten. Vor und nach den genannten Stufen können noch weitere Temperaturzonen vorliegen.

Erfindungsgemäß können alle sauerstoffhaltigen Gase in Schritt (D) des erfindungsgemäßen Verfahrens eingesetzt werden, die der Fachmann für geeignet hält, beispielsweise Luft, mit Sauerstoff angereicherte Luft oder Sauerstoff, gegebenenfalls in Mischung mit inerten Gasen wie Stickstoff oder Edelgase wie Helium oder Argon. In einer bevorzugten Ausführungsform wird Luft als sauerstoffhaltiges Gas in Schritt (D) des erfindungsgemäßen Verfahrens eingesetzt.

Unter "Luft" wird im Rahmen der vorliegenden Erfindung eine Mischung verstanden, enthaltend 70 bis 80 Gew.-%, bevorzugt 73 bis 77 Gew.-%, Stickstoff, 20 bis 30 Gew.-%, bevorzugt 21 bis 26 Gew.-%, Sauerstoff und kleinere Mengen, d. h. unterhalb 1,5 Gew.-%, Kohlendioxid, Kohlenmonoxid und/oder Edelgase.

Es ist erfindungsgemäß möglich, dass vor Schritt (D) das in Schritt (C) erhaltene Oxidgemisch in eine für die spätere Verwendung als Katalysator geeignete Form, beispielsweise Hohlstränge, Ringe, Tabletten, Ringtabletten, Sterne, Hohlsterne, gebracht wird. Es ist erfindungsgemäß auch möglich, dass das nach Schritt (D) erhaltene Oxidgemisch in die für die Verwendung als Katalysator geeignete Form gebracht wird. Es ist auch möglich, dass das in Schritt (C) erhaltene Oxidgemisch in Schritt (D) kalziniert wird, dann in Form gebracht wird, und anschließend wieder gemäß Schritt (D) kalziniert wird.

Es ist auch möglich, dass bereits in Schritt (D) kalziniertes Oxidgemisch mit nicht kalziniertem Oxidgemisch gemischt wird, und diese Mischung in Schritt (D) kalziniert wird, wobei die Formgebung vor oder nach dem Kalzinieren stattfinden kann.

Das Formgeben des erfindungsgemäßen Oxidgemisches ist dem Fachmann im Allgemeinen bekannt.

In einer ersten Ausführungsform wird das erfindungsgemäß hergestellte Oxidgemisch zusammen mit dem Fachmann bekannten Additiven bzw. Zusatzstoffen in Form gebracht. Geeignete Zusatzstoffe sind beispielsweise Hilfsstoffe, die die Verarbeitbarkeit, die mechanische Festigkeit oder die Porenstruktur verbessern. Beispiele für solche Stoffe sind Kartoffelstärke, Zellulose, Stearinsäure, Graphit und/oder Portland-Zement. Diese Einsatzstoffe können direkt in einen Mischer, Kneter oder Mix-Muller mit dem Oxidgemisch, beispielsweise bei der Formgebung, vermischt werden. Ferner können die Einsatzstoffe auch in einer Füllmaschine mit dem Oxidgemisch gemeinsam aufgeschlämmt und in einem Sprühtrockner zu einem Sprühpulver verarbeitet werden. Die Einsatzstoffe werden vorzugsweise in einem Mix-Muller oder Kneter mit dem Oxidgemisch unter Zugabe von Wasser zu einer extrudierbaren, pasteusen Masse verarbeitet.

Die vorliegende Erfindung betrifft auch ein Oxidgemisch enthaltend wenigstens eine Verbindung der allgemeinen Formel (I)

Feₓ(M¹O_{y})_{z} (I)

und wenigstens eine Verbindung der allgemeinen Formel (II)

M¹ₘOₙ (II),

mit den oben definierten Bedeutungen für M¹, x, y, z, m und n, herstellbar nach dem oben beschriebenen Verfahren.

In einer bevorzugten Ausführungsform liegt in der Verbindung der allgemeinen Formel (I) Fe in der Oxidationsstufe +3 vor. In einer besonders bevorzugten Ausführungsform liegt in dem erfindungsgemäßen Oxidgemisch kein, d. h. weniger als 0,5 Gew.-%, Eisen in der Oxidationsstufe +2, beispielsweise als FeMoO₄, vor.

Des Weiteren betrifft die vorliegende Erfindung auch die Verwendung des erfindungsgemäß herstellbaren Oxidgemisches als Katalysator bei der Herstellung von Formaldehyd durch Oxidation von Methanol.

Die vorliegende Erfindung betrifft auch ein Verfahren zur Herstellung von Formaldehyd durch Oxidation von Methanol, wobei ein erfindungsgemäß herstellbares Oxidgemisch als Katalysator eingesetzt wird.

Das erfindungsgemäße Verfahren der Methanol-Oxidation ist dem Fachmann bekannt und wird im Allgemeinen Formox-Verfahren genannt. Dabei wird im Allgemeinen Formaldehyd aus Methanol bei einer Temperatur von 200 bis 400°C, bevorzugt 220 bis 320°C in einem Druck von 1 bis 10 bar, bevorzugt 1 bis 3 bar, erhalten. Das Verfahren kann in einem beliebigen Festbett-Reaktor, beispielsweise einem Rohr- oder Rohrbündel-Reaktor, oder einem Reaktor der in WO 2005/063375 beschriebenen Art durchgeführt werden.

Die Erfindung wird durch die nachstehenden Beispiele näher erläutert.

### Beispiele

Herstellung eines erfindungsgemäßen Sprühpulvers:
530 g Ammoniumheptamolybdat (M = 1236 g/mol; (NH₄)₆Mo₇O₂₄ · 4H₂O) werden in 800 ml VE-Wasser und 290 g 25 gew.-%igem Ammoniakwasser bei 60 °C gelöst. In diese Lösung wird eine Lösung von 808 g Eisen(III)nitrat (M = 404 g/mol, Fe(NO₃)₃ · 9H₂O) in 1 L VE-Wasser über einen Zeitraum von 20 Minuten bei 60 °C eingerührt. Es wird noch 5 Minuten bei 60 °C nachgerührt. Nach Sprühtrocknung wird ein Pulver erhalten, welches weiterverarbeitet wird.
30 Gew.-% kalziniertes Sprühpulver SPKZ und 70 Gew.-% nicht kalziniertes Sprühpulver SPLV werden gemischt, und diese Mischung wird mit 3 Gew.-% Graphit gemischt, kompaktiert, und dann zu Ringtabletten mit den Abmessungen 5 mm · 3 mm · 2 mm geformt.

Die erfindungsgemäß hergestellten Katalysatoren 1, 2, 3, und 4 werden anschließend in Luft bei einer Temperatur von 480 °C calciniert. Dabei werden die Katalysatoren zunächst 10 Stunden bei 120 °C kalziniert, dann wird die Temperatur innerhalb 10 Stunden auf 460 °C gesteigert, und abschließend 4 Stunden bei 460 °C gehalten. Die Kalzinierung wird mit 25 (Kat. 1), 50 (Kat. 2), 75 (Kat. 3) oder 100 NI/h (Kat. 4) durchgeführt.

### Katalysator 5:

Nicht kalziniertes Sprühpulver SPLV wird mit 3 Gew.-% Graphit gemischt, kompaktiert, und dann zu Ringtabletten mit den Abmessungen 5 mm · 3 mm · 2 mm geformt. Die Kalzinierung wird mit 100 NI/h durchgeführt.

### Katalysator 6:

25 Gew.-% kalziniertes Sprühpulver SPKZ und 75 Gew.-% nicht kalziniertes Sprühpulver SPLV werden gemischt, und diese Mischung wird mit 3 Gew.-% Graphit gemischt, kompaktiert, und dann zu Ringtabletten mit den Abmessungen 5 mm · 3 mm · 2 mm geformt. Die Kalzinierung wird mit 100 NI/h durchgeführt.

### Katalysator 7:

30 Gew.-% kalziniertes Sprühpulver SPKZ und 70 Gew.-% nicht kalziniertes Sprühpulver SPLV werden gemischt, und diese Mischung wird mit 3 Gew.-% Graphit gemischt, kompaktiert, und dann zu Ringtabletten mit den Abmessungen 5 mm · 3 mm · 2 mm geformt. Die Kalzinierung wird mit 100 NI/h durchgeführt.

Die einzelnen Kalzinierungen der Katalysatoren 1, 2, 3, 4 und 5 bis 7 unterscheiden sich von einander u. a. durch die unterschiedlichen Flussraten der Luft, und somit auch durch das molare Verhältnis von Sauerstoff zu NH₄NO₃, siehe Tabelle 1

**Tabelle 1**

| Katalysator Nr. | 1 | 2 | 3 | 4 | 5 | 6 | 7 |
|---|---|---|---|---|---|---|---|
| Flussrate Luft [NI/h] | 25 | 50 | 75 | 100 | 100 | 100 | 100 |
| n(O₂)/n(NH₄NO₃) | 0,04 | 0,1 | 0,2 | 0,3 | 0,3 | 0,3 | 0,3 |

Die so erhaltenen Katalysatoren 1, 2, 3, 4, 5, 6 und 7 werden bei der Herstellung von Formaldehyd aus Methanol mit einer GHSV (Gas Hourly Space Velocity) von 6500 m³ Gas/m³ Katalysator eingesetzt. Das gasförmige Substrat enthält 3 Vol.-% Wasser, 9 Vol.-% Methanol, Rest Stickstoff. Das molare Verhältnis von Methanol zu Sauerstoff n(MeOH)/n(O₂) beträgt 0,91. Die Reaktion wird bei einer Temperatur von 265 bis 280 °C und einem Druck von 320 mbar in einem Salzbad -Reaktor für eine Reaktionsdauer von mehreren Tagen durchgeführt. Es werden der Umsatz bezüglich Methanol, die Ausbeute und die Selektivität bezüglich Formaldehyd bestimmt. Die Ergebnisse der einzelnen Versuche sind in Tabelle 2 dargestellt.

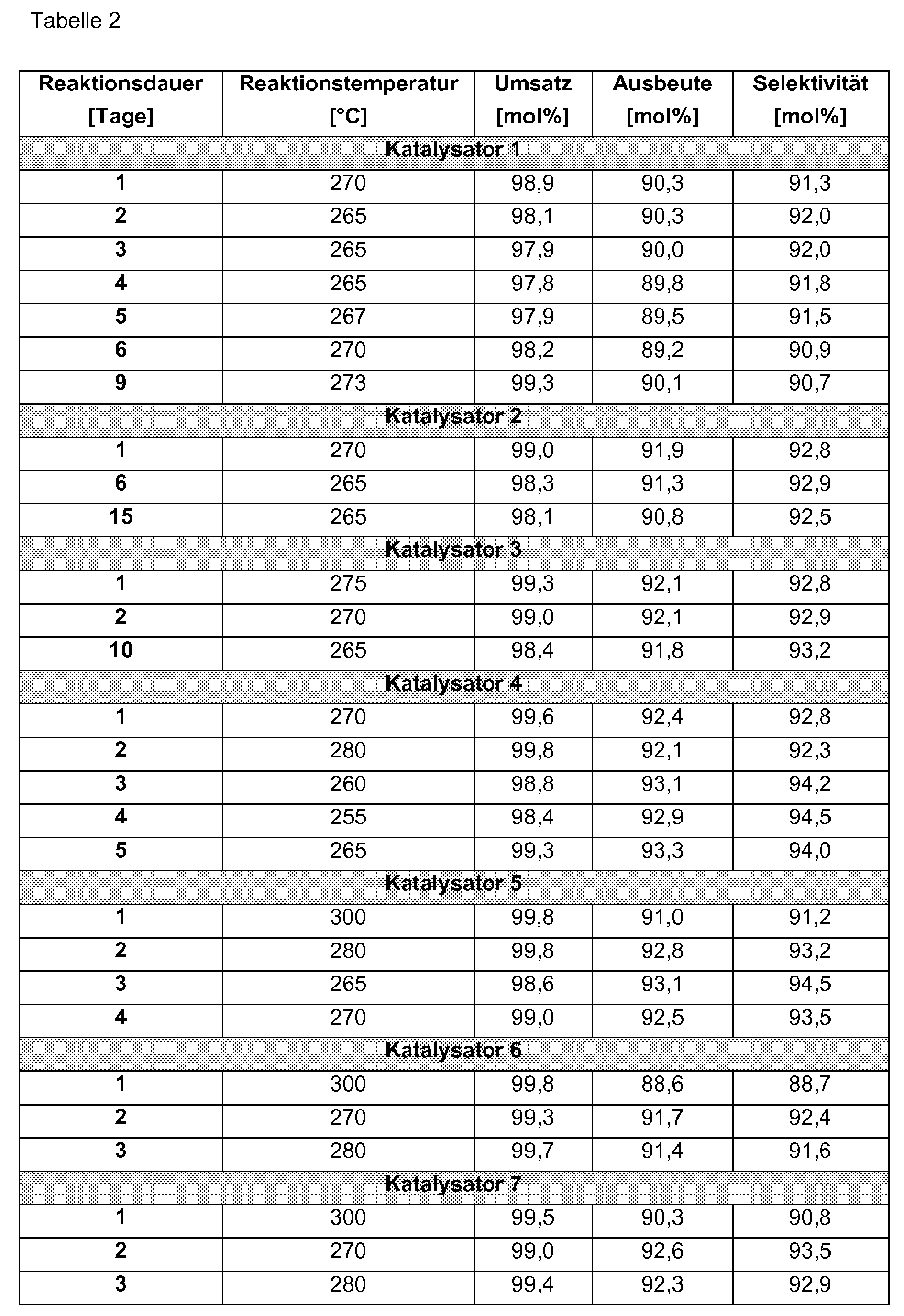

Die Mengen an Methanol, Formaldehyd und dem Nebenprodukt Kohlendioxid werden durch Gaschromatographie (Methanol), Titration (Formaldehyd) und IR-Spektroskopie (Kohlendioxid) bestimmt. Diese Verfahren sind dem Fachmann bekannt. Aus den erhaltenen Werten lassen sich Umsatz, Ausbeute und Selektivität bestimmen.

Die in Tabelle 2 gezeigten Versuchsergebnisse zeigen, dass die Flussrate im Calcinierungsschritt bei der Herstellung des Katalysators einen entscheidenden Einfluss auf Umsatz, Ausbeute und Selektivität der Umsetzung von Methanol zu Formaldehyd hat. Die erfindungsgemäß hergestellten Katalysatoren zeigen auch über lange Reaktionsdauern gleich bleibend gute Ergebnisse, weisen somit eine hohe Stabilität unter den gegebenen Versuchsbedingungen.

## Patentansprüche

1. Verfahren zur Herstellung eines Oxidgemisches enthaltend wenigstens eine Verbindung der allgemeinen Formel (I)
Feₓ(M¹O_{y})_{z} (I)
und wenigstens eine Verbindung der allgemeinen Formel (II)
M¹ₘOₙ (II),
in denen M¹, x, y, z, m und n die folgenden Bedeutungen haben:
M¹ Metall ausgewählt aus der Gruppe bestehend aus Mo, V, Cu, Cr, Co, P, Si, B, S, Na und Mischungen davon,
x ganze Zahl von 1 bis 3,
y ganze Zahl von 1 bis 6,
z ganze Zahl von 1 bis 4,
m ganze Zahl von 1 bis 3 und
n ganze Zahl von 1 bis 5,
wobei x, y, z, m und n so gewählt sind, dass die Verbindungen der allgemeinen Formeln (I) und (II) jeweils ladungsneutral sind, umfassend die Schritte:
(A) Bereitstellen einer Lösung von Verbindungen enthaltend wenigstens Eisen- und M¹-Kationen in einem geeigneten Lösungsmittel,
(B) Herstellen eines Oxidgemisches enthaltend wenigstens eine Verbindung der allgemeinen Formel (I) und wenigstens eine Verbindung der allgemeinen Formel (II) durch gemeinsames Ausfällen der Verbindungen aus der in Schritt (A) hergestellten Lösung,
(C) Abtrennen des in Schritt (B) erhaltenen Oxidgemisches von dem Lösungsmittel und
(D) Calcinieren des in Schritt (C) erhaltenen Oxidgemisches bei einer Temperatur von 80 bis 550 °C in einem sauerstoffhaltigen Gas,
**dadurch gekennzeichnet, dass** sich aus den in Schritt (A) eingesetzten Verbindungen in Schritt (B) und/oder (D) wenigstens eine Verbindung G bildet, die von den Verbindungen der allgemeinen Formeln (I) und (II) unterschiedlich ist, und in Schritt (D) das molare Verhältnis von Sauerstoff zu der wenigstens einen Verbindung G n(O₂)/n(G) größer als 0,10 ist.

2. Verfahren nach Anspruch 1, **dadurch gekennzeichnet, dass** die Verbindung der allgemeinen Formel (I) Fe₂(MoO₄)₃ ist, und die Verbindung der allgemeinen Formel (II) MoO₃ ist.

3. Verfahren nach Anspruch 1 oder 2, **dadurch gekennzeichnet, dass** das Lösungsmittel in Schritt (A) Wasser ist.

4. Verfahren nach einem der Ansprüche 1 bis 3, **dadurch gekennzeichnet, dass** die in Schritt (A) hergestellte Lösung Fe(NO₃)₃ und wenigstens ein Ammoniummolybdat enthält.

5. Verfahren nach Anspruch 4, **dadurch gekennzeichnet, dass** die Verbindung G NH₄NO₃ ist.

6. Verfahren nach einem der Ansprüche 1 bis 5, **dadurch gekennzeichnet, dass** das Abtrennen in Schritt (C) durch Sprühtrocknen erfolgt.

7. Verfahren nach einem der Ansprüche 1 bis 6, **dadurch gekennzeichnet, dass** das sauerstoffhaltige Gas in Schritt (D) Luft ist.

8. Oxidgemisch enthaltend wenigstens eine Verbindung der allgemeinen Formel (I)
Feₓ(M¹O_{y})_{z} (I)
und wenigstens eine Verbindung der allgemeinen Formel (II)
M¹ₘOₙ (II),
mit den in Anspruch 1 definierten Bedeutungen für M¹, x, y, z, m und n, herstellbar nach dem Verfahren gemäß einem der Ansprüche 1 bis 7.

9. Verwendung des Oxidgemisches gemäß Anspruch 8 als Katalysator bei der Herstellung von Formaldehyd durch Oxidation von Methanol.

10. Verfahren zur Herstellung von Formaldehyd durch Oxidation von Methanol, **dadurch gekennzeichnet, dass** ein Oxidgemisch gemäß Anspruch 8 als Katalysator eingesetzt wird.
